# DEMANDE DE BREVET EUROPEEN

(11) **EP 0 571 295 A1**
(43) Date de publication de la demande: **24.11.1993**
(21) Numéro de dépôt: 93401313.7
(22) Date de dépôt: 21.05.1993
(51) Int. Cl.: G01N 33/28, G01N 33/30

(54) **Dispositif de cotation d'un lubrifiant, notamment de moteur de véhicule automobile**

(30) Priorité: 21.05.1992 FR 9206200
(71) Demandeur: A.D.D.S. CONCEPT, F-76320 Caudebec les Elbeuf (FR)
(72) Inventeur: Delannoy, Alain, F-76320 Caudebec les Elbeuf (FR)
(74) Mandataire: Habasque, Etienne Joel Jean-François

(57) **Abrégé**

La demande concerne un dispositif de cotation d'un lubrifiant, notamment de moteur de véhicule automobile. Ce dispositif est caractérisé en ce qu'il comporte :
- des moyens (1) de prise d'une image en couleurs d'un dépôt normalisé de lubrifiant sur la surface d'une éprouvette (E),
- des moyens (2) de numérisation de cette image,
- des moyens (3) d'analyse de cette image numérisée, portion par portion, à l'aide d'une charte de couleurs normalisée (4), pour établir une table (5) de couleurs contenant, portion d'image par portion d'image, des informations de répartition des différentes couleurs de la charte, et
- des moyens (6) de cotation du lubrifiant à partir des informations de couleurs de la table.

## Description

La présente invention concerne un dispositif de cotation d'un lubrifiant, notamment de moteur de véhicule automobile.

Ce dispositif est destiné à être utilisé par exemple par des fabricants d'additifs, des raffineurs, ou encore des motoristes, qui mettent au point très fréquemment des nouveaux lubrifiants qu'il y a lieu de coter selon des normes bien établies, par exemple par l'Institut Français du Pétrole (IFP), pour déterminer la qualité de ces lubrifiants.

Pour réaliser cette cotation de lubrifiant, il existe dans l'état de la technique un certain nombre de contrôles.

Parmi ceux-ci, on peut citer :
- Le contrôle de dispersion,
- Le contrôle de microcokage,
- Le contrôle du PCT (Panel cooking Test), et
- Le contrôle des pistons, par exemple d'un moteur.

Les trois premiers contrôles mentionnés précédemment sont des contrôles dits statiques tandis que le quatrième contrôle est un contrôle dit dynamique.

Ces différents contrôles sont également normalisés et bien connus des spécialistes du domaine en question de sorte que l'on ne les décrira pas en détail.

Les méthodes de cotation actuellement en vigueur sont des méthodes de cotation visuelles de ces lubrifiants et consistent, pour un spécialiste, à analyser un dépôt normalisé de lubrifiant sur une éprouvette, portion par portion, pour déterminer, à l'aide d'une charte de couleurs normalisée, la répartition des différentes couleurs de cette charte dans les différentes portions de l'éprouvette, pour tirer de cette analyse une cotation du lubrifiant.

On conçoit que ces méthodes de cotation visuelles sont très longues, difficiles, demandent une formation de personnel extrêmement pointue et ne permettent pas une cotation fiable et rapide.

Il a déjà été développé dans l'état de la technique, des dispositifs de cotation automatiques qui utilisent une caméra en noir et blanc et qui permettent de réaliser une cotation par le contrôle de dispersion.

Cependant, l'utilisation de ces dispositifs est relativement limitée car ils ne permettent de réaliser qu'un seul contrôle.

Le but de l'invention est donc de résoudre ces problèmes en proposant un dispositif de cotation automatique d'un lubrifiant qui puisse être utilisé pour mettre en oeuvre les différents contrôles normalisés permettant de coter ce lubrifiant.

A cet effet, l'invention a pour objet un dispositif de cotation d'un lubrifiant, notamment de moteur de véhicule automobile, caractérisé en ce qu'il comporte :
- des moyens de prise d'une image en couleurs d'un dépôt normalisé du lubrifiant sur la surface d'une éprouvette,
- des moyens de numérisation de cette image,
- des moyens d'analyse de cette image numérisée, portion par portion, à l'aide d'une charte de couleurs normalisée, pour établir une table de couleurs contenant, portion d'image par portion d'image, des informations de répartition des différentes couleurs de la charte, et
- des moyens de cotation du lubrifiant à partir des informations de couleurs de la table.

Avantageusement, ce dispositif comporte également des moyens de mesure de l'épaisseur du dépôt de lubrifiant sur l'éprouvette.

L'invention sera mieux comprise à l'aide de la description qui va suivre, donnée uniquement à titre d'exemple et faite en se référant aux dessins annexés sur lesquels :
- La figure 1 représente un schéma synoptique d'un mode de réalisation d'un dispositif de cotation selon l'invention, et
- Les figures 2 à 7 illustrent les différentes étapes de fonctionnement du dispositif représenté sur la figure 1.

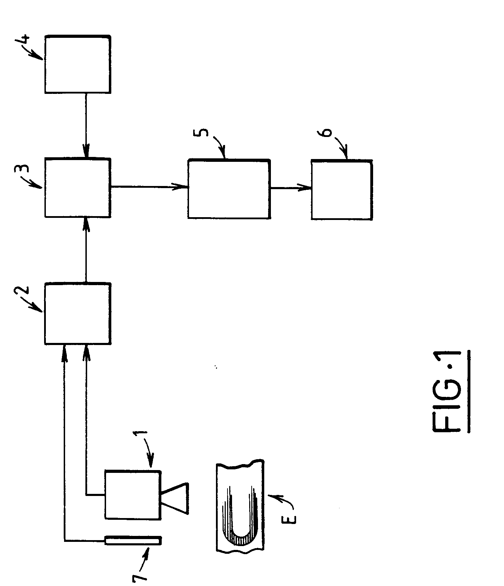

Ainsi qu'on peut le voir sur ces figures et plus particulièrement sur la Fig.1, le dispositif de cotation selon l'invention comporte des moyens désignés par la référence générale 1 permettant de prendre une image en couleurs d'un dépôt normalisé d'un lubrifiant sur la surface d'une éprouvette E.

Ainsi qu'on l'a indiqué précédemment, ces dépôts normalisés de lubrifiant sont régis de manière extrêmement précise par différentes normes établies par exemple par l'Institut Français du Pétrole et permettent de réaliser des cotations de lubrifiant par contrôle de microcokage, du PCT (Panel Cooking Test) ou des pistons, par exemple d'un moteur.

En fait, ces différents contrôles sont déjà mis en oeuvre dans l'état de la technique mais la cotation se fait visuellement par un opérateur.

Le but de l'invention est de remplacer cette cotation visuelle par une cotation automatique à partir des mêmes dépôts sur éprouvette.

Une éprouvette sur laquelle du lubrifiant a été déposé selon l'une ou l'autre des méthodes indiquées précédemment est placée en regard de ces moyens de prise d'une image.

En fait, ces moyens peuvent être constitués par tout organe approprié permettant de prendre une image en couleurs de ce dépôt.

La sortie des ces moyens de prise d'image est reliée à l'entrée de moyens de numérisation désignés par la référence générale 2 et constitués par exemple par toute carte de numérisation appropriée.

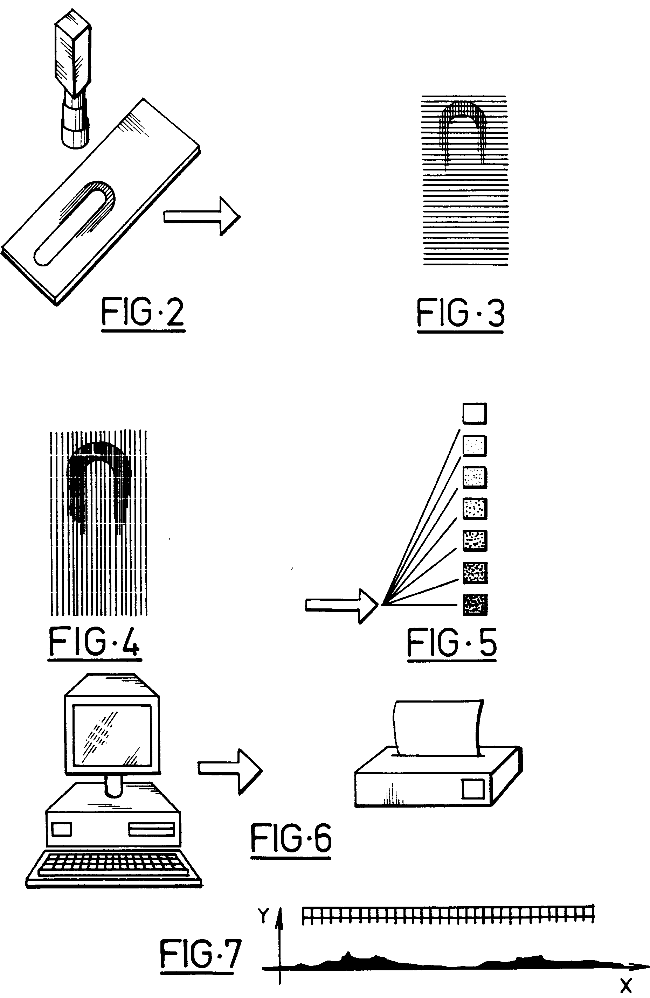

Ces étapes sont illustrées sur les figures 2 et 3.

La sortie de ces moyens de numérisation est reliée à l'entrée de moyens 3 d'analyse de cette image numérisée portion par portion, à l'aide d'une charte de couleurs normalisée, également établie par l'Institut Français du Pétrole, pour établir une table de couleurs contenant, portion d'image par portion d'image, des informations de répartition des différentes couleurs de cette charte (Fig.4 et 5).

Cette charte de couleurs normalisée est par exemple stockée dans des moyens de mémorisation 4 connectés à ces moyens d'analyse 3. La table de couleurs est également stockée dans des moyens de mémorisation 5 reliés aux moyens d'analyse 3.

En fait, ces moyens d'analyse peuvent être constitués par tout calculateur approprié permettant d'effectuer une comparaison des couleurs de chaque portion de l'image à une palette de couleurs de la charte allant du noir au blanc en passant par quelques nuances de marron.

L'analyse de cette image permet donc de remettre toutes les couleurs analysées de chaque portion d'image dans des niveaux correspondants de la charte.

L'analyse de l'image peut être réalisée à l'aide d'une grille d'analyse normalisée, également établie par l'Institut Français du Pétrole, cette grille étant superposée à l'image pour permettre l'analyse de celle-ci portion par portion.

Dans ce cas particulier, cette grille peut être une grille virtuelle établie par programmation des moyens d'analyse 3.

Une fois la carte de couleurs établie et stockée dans les moyens de mémorisation 5, il est alors possible à des moyens de cotation 6 connectés à ces moyens de mémorisation, d'opérer la cotation, c'est-à-dire la notation du lubrifiant, à partir des différentes informations de répartition de couleurs de cette table (Fig.6).

Ces moyens de cotation peuvent en fait être constitués par un calculateur neuronal dont le fonctionnement est établi après une phase d'apprentissage.

Ainsi, après une phase d'apprentissage et de configuration de ce calculateur, celui-ci est apte à réaliser une cotation de façon automatique.

Ce calculateur neuronal peut en fait être un calculateur multicouche comportant une couche de neurones d'entrée, au moins une couche cachée de neurones et une couche de neurones de sortie.

Par ailleurs, ce calculateur est de préférence un calculateur à rétropropagation du gradient.

En fait, ce calculateur peut être constitué par tout calculateur approprié dans lequel est chargé un logiciel du type NEURALWORKS, commercialisé par la Société NEURALWARE, ce logiciel incorporant le principe de rétropropagation du gradient et étant appliqué aux modèles multicouches du type PERCEPTRON.

Les neurones définis dans ce type de logiciels, sont les éléments de base d'un système central et chacun d'eux assure plusieurs fonctions :
- de réception des signaux provenant des neurones voisins ;
- d'intégration de ces signaux pour engendrer un signal de sortie; et
- de transmission de ce signal de sortie vers un neurone voisin.

Chaque neurone formel effectue en fait une sommation, pondérée par des poids, des valeurs qui arrivent d'autres neurones par exemple, puis réalise une corrélation du résultat de cette sommation avec une fonction de seuil, avant d'assurer sa transmission vers d'autres neurones.

Le problème majeur de ce type de réseau neuronal consiste à déterminer les poids des connexions entre ces neurones.

Les neurones formels sont organisés en couches dans le dispositif selon l'invention dans la mesure où cette organisation est imposée par la méthode de rétropropagation du gradient permettant de calculer les poids mentionnés précédemment.

Chaque couche est composée de neurones recevant uniquement des signaux provenant de neurones de la couche précédente et émet des signaux uniquement vers des neurones de la couche suivante.

Aucune connexion intracouche n'est permise.

On notera que la fonction de seuil utilisée par la méthode de rétropropagation du gradient doit être continue et est généralement une sigmoïde.

La méthode de rétropropagation du gradient est une méthode bien connue dans l'état de la technique et ne sera donc pas exposée dans le détail.

Cette méthode permet de résoudre l'un des principaux problèmes des réseaux de neurones, qui est la détermination du poids à attribuer à chaque connexion.

La mise en oeuvre d'un calculateur neuronal de ce type, commence par une phase d'apprentissage de celui-ci.

Cette phase d'apprentissage se décompose comme suit :
- fonctionnement du réseau en reconnaissance pour obtenir un résultat en fonction d'une entrée. Dans ce mode de fonctionnement, le calcul se réalise de l'entrée vers la sortie (propagation) ;
- comparaison de ce résultat avec celui que l'on aurait voulu obtenir, puis réajustement des poids des connexions afin de se rapprocher de la sortie désirée. Le calcul s'effectue dans ce cas de la sortie vers l'entrée et on dit que l'on rétropropage l'erreur.

Ces deux étapes sont répétées autant de fois qu'il est jugé nécessaire sur des exemples différents.

Ainsi, la méthode d'apprentissage utilisée est celle de la rétropropagation du gradient qui réajuste les poids des connexions entre les neurones en fonction de l'erreur commise.

Cet apprentissage du calculateur neuronal étant bien connu dans l'état de la technique, et ne présentant pas de difficultés particulières, on ne le décrira pas plus en détail par la suite.

Ainsi ce calculateur est configuré pour obtenir de façon automatique une cotation qui était obtenue de façon manuelle et visuelle par un opérateur, dans l'état de la technique.

On conçoit que plus la définition et la précision de la chaîne d'analyse de l'image du dépôt sont bonnes, plus la cotation est précise.

Il va de soi que l'analyse de l'image, peut également être réalisée élément d'image par élément d'image sans utiliser la grille normalisée de l'Institut Français du Pétrole, ce qui permet encore d'améliorer les performances de cotation.

Comme on peut le voir sur la figure 1, le dispositif selon l'invention peut également comporter des moyens de mesure de l'épaisseur du dépôt.

Ces moyens de mesure sont désignés par la référence générale 7 sur cette figure et comprennent par exemple des moyens de mesure à faisceau laser, reliés aux moyens de numérisation 2 décrits précédemment pour intégrer dans la table mentionnée précédemment, des informations d'épaisseur de dépôt (Fig.7). Ces informations sont alors également prises en compte par le calculateur 6 pour côter le lubrifiant.

Ceci permet de réaliser une cotation selon le contrôle du PCT (Panel Cooking Test).

L'éprouvette et/ou les moyens de mesure sont alors montés déplaçables l'un par rapport à l'autre de façon classique pour assurer un balayage du dépôt et une mesure de l'épaisseur de celui-ci sur la totalité de l'éprouvette.

Ces informations d'épaisseur de dépôt sont également stockées dans les moyens de mémorisation 5 et analysées pour obtenir la cotation définitive du lubrifiant par le calculateur 6.

On conçoit que le dispositif de cotation selon l'invention peut être utilisé pour mettre en oeuvre tous les contrôles mentionnés précédemment et en particulier les contrôles de microcokage, du PCT (Panel Cooking Test) et de piston.

On rappellera que ce dernier contrôle consiste à analyser les dépôts de lubrifiant sur un piston de moteur ayant fonctionné selon des régimes bien établis et normalisés.

Enfin, les moyens d'analyse 3 et les moyens de cotation 6 peuvent être intégrés dans la même unité de traitement d'informations à base de microprocesseur associée à des mémoires de stockage d'informations pour la mémorisation de la charte de couleurs et de la table de couleurs obtenue après analyse d'une image.

On conçoit donc que le dispositif de cotation selon l'invention peut être utilisé pour réaliser la cotation d'un lubrifiant selon l'un ou l'autre des contrôles indiqués précédemment et ceci de façon automatique et extrêmement fiable.

Bien entendu, le dispositif de cotation selon l'invention peut être utilisé pour côter des lubrifiants utilisables dans des domaines autres que l'automobile, tels que par exemple l'aviation, etc...

## Revendications

**1.-** Dispositif de cotation d'un lubrifiant, notamment de moteur de véhicule automobile, caractérisé en ce qu'il comporte :
- des moyens (1) de prise d'une image en couleurs d'un dépôt normalisé de lubrifiant sur la surface d'une éprouvette (E),
- des moyens (2) de numérisation de cette image,
- des moyens (3) d'analyse de cette image numérisée, portion par portion, à l'aide d'une charte de couleurs normalisée (4), pour établir une table (5) de couleurs contenant, portion d'image par portion d'image, des informations de répartition des différentes couleurs de la charte, et
- des moyens (6) de cotation du lubrifiant à partir des informations de couleurs de la table.

**2.-** Dispositif de cotation d'un lubrifiant selon la revendication 1, caractérisé en ce qu'il comporte des moyens (7) de mesure de l'épaisseur du dépôt de lubrifiant sur l'éprouvette (E), ces informations d'épaisseur de dépôt étant prises en compte par les moyens de cotation (6) pour réaliser la cotation du lubrifiant.

**3.-** Dispositif de cotation d'un lubrifiant selon l'une des revendications 1 ou 2, caractérisé en ce que les moyens de cotation (6) comprennent un calculateur neuronal.
